# EUROPEAN PATENT SPECIFICATION

(11) **EP 1 572 440 B1**
(45) Date of publication and mention of the grant of the patent: **08.12.2010**
(21) Application number: 03797016.7
(22) Date of filing: 15.12.2003
(51) Int. Cl.: B29D 11/02, B29D 11/00, A61F 2/16

(54) **APPARATUS AND METHOD FOR CUTTING AN INTRAOCULAR LENSE FROM A BLANK**
GERÄT UND VERFAHREN ZUM SCHNEIDEN EINER INTRAOKULARLINSE AUS EINEM ROHLING
EMPORTE-PIECE A BORD DROIT IOL ET APPAREIL D'INSERTION HAPTIQUE

(30) Priority: 20.12.2002 US 327580; 26.06.2003 US 606553
(43) Date of publication of application: 14.09.2005
(73) Proprietor: BAUSCH & LOMB INCORPORATED, Rochester, New York 14604-2701 (US)
(72) Inventor: HOVEY, Larry, C., Ontario, NY 14519 (US); REED, Brian, Penfield, New York 14526 (US); APPLETON, William J., Rochester, NY 14622 (US); O'CALLAGHAN, James, Carrick-on-Sur, Waterford (IE); URBANOWICZ, Tadesz, Rochester, New York 14625 (US); SNOW, Larnese, Las Flores, California 92688 (US); SUBRIN, Philippe, CH-3076, Worb (CH); ADAMS, Bradley J., Newport Richey, Florida 34653 (US)
(74) Representative: Carpmael, Robert Maurice Charles
(86) International application number: PCT/US2003/039800
(87) International publication number: WO 2004/060216

(56) References cited:
- EP-A- 0 897 777
- GB-A- 556 743
- US-A- 4 813 956
- US-A- 5 071 101
- US-A- 5 611 968
- US-A- 5 762 836
- PATENT ABSTRACTS OF JAPAN vol. 1997, no. 03, 31 March 1997 (1997-03-31) -& JP 08 304747 A (MENICON CO LTD), 22 November 1996 (1996-11-22)
- PATENT ABSTRACTS OF JAPAN vol. 018, no. 276 (C-1204), 26 May 1994 (1994-05-26) -& JP 06 048749 A (OLYMPUS OPTICAL CO LTD), 22 February 1994 (1994-02-22)

## Description

### Related Application

This application is a continuation-in-part of prior application serial number 10/327,580 filed on December 20, 2002.

### Background of the Invention

The present invention relates to the manufacture of intraocular lenses (hereinafter IOLs). More particularly, the invention relates to a fixture and method for cutting an IOL optic with a square edge and subsequently attaching one or more haptics to the IOL optic.

A common and desirable method of treating a cataract eye is to remove the clouded, natural lens and replace it with an artificial IOL in a surgical procedure known as cataract extraction. IOLs are available in many different configurations and materials which the surgeon chooses from based on the needs of the patient. Some of the more common IOLs include an optic and one or more but usually two haptics extending from the optic for anchoring the IOL within the eye. The IOL optic may itself be bi-convex, plano-convex, plano-concave, plano-plano, or bi-concave, for example. The optic may also include spheric and/or aspheric optics on one or more surfaces thereof. Materials from which IOLs are made include silicone, silicone acrylates, hard and soft acrylics, for example. The haptics may be of the same or different material from which the optic is formed. Presently popular IOL designs have a flexible optic formed of silicone elastomer or soft acrylic, for example, while the haptics are formed from a more rigid material such as PMMA which is a hard acrylic, for example. A flexible optic portion is desirable so that the optic may be folded and/or compressed for delivery through a relatively small incision made in the eye (e.g., about or less than 3mm). Once in the eye, the optic resumes its original, unstressed shape. More rigid haptics are desirable so that they may function to locate and stabilize the optic within the eye. The haptics may be formed integrally with the optic or attached to the optic after the optic is formed. An example of co-molding the optic and haptics together where the optic is formed from a flexible material and the haptics are formed from a rigid material may be seen in U. S. Pat. Nos. 5,217,491 and 5,326,506 to Vanderbilt. The resultant rod of composite material is then machined (e.g., milled or lathed) into the final IOL shape including both the optic and haptic portions thereof. US Patent number 6,045,577 discloses an ophthalmic lens with reduced edge glare

Various methods for making IOLs have been proposed in the prior art, with molding and milling/lathing being the most popular. Japan patent publication number JP 6048749 (A) discloses a device and method for the production of ring-shaped lenses.

### Summary of the Invention

The present invention is defined in accordance with accompanying claims 1 and 9. The invention provides an apparatus and method for making an IOL having an optic with a square edge and subsequent attachment of one or more haptics to the optic. The apparatus comprises a fixture which incorporates a cutting implement (e.g., a trephine) for cutting the IOL optic from a blank material of silicone, for example. The IOL blank is preferably molded and then polished for a smooth surface. With the IOL blank appropriately mounted to the fixture, a trephine cuts the blank to form the IOL optic peripheral edge which requites no further polishing as is required in many of the prior optic forming methods. The trephine may be mounted within a fixture having a work piece holder upon which the blank material may be positioned for cutting. The blank material is moved relative to the trephine in a simultaneous rotating and linear translation to make the cut. The blank is preferably supplied in a disc form with the trephine cutting the disc at a location which is radially inward of the outer periphery of the disc. As such, the trephine cuts the desired optic diameter while leaving an outer ring of material which is discarded or recycled. The trephine is preferably removable from the fixture such that it may be quickly and easily replaced when the blade thereof is worn, or when it is desired to change to a trephine having a different optic cutting diameter.

The resultant optic is formed with a straight peripheral wall that extends substantially parallel to the optical axis of the IOL optic. At least the juncture of the optic posterior surface and peripheral wall form a sharp peripheral edge which has been clinically shown to help reduce the occurrence of posterior capsular opacification (PCO) or secondary cataracts.

The fixture may also include an optic release mechanism for lifting the cut optic from the fixture for easy retrieval thereof with a pair of tweezers, for example.

In an aspect of the invention, a method for cutting an IOL optic having opposite anterior and posterior surfaces and a peripheral wall extending therebetween out of an IOL blank, the method comprising the steps of:
a) providing a generally circular cutting edge;
b) providing a lens press having a generally circular lens-engaging end;
c) positioning said IOL blank between said generally circular cutting edge and said generally circular lens-engaging end of said lens press;
d) moving said lens press and said cutting edge toward one another in a rotational translation with said cutting edge rotationally cutting through said IOL blank and thereby forming said IOL optic.

In a further aspect of the invention, an apparatus for cutting an IOL optic opposite anterior and posterior surfaces and a peripheral wall extending therebetween out of an IOL blank, said apparatus comprising:
a) a generally circular cutting edge;
b) a lens press having a generally circular lens-engaging end with said IOL blank positionable between said generally circular cutting edge and said generally circular lens-engaging end of said lens press;
wherein said IOL optic is formed by moving said lens press and said cutting edge toward one another in a rotational translation with said cutting edge rotationally cutting through said IOL blank and thereby forming said IOL optic.

The apparatus and method are for cutting an intraocular lens having an optic portion with opposite anterior and posterior surfaces and an outer peripheral wall extending therebetween, the juncture of the peripheral wall and the posterior surface forming a sharp edge, with the peripheral wall optionally including generally helically shaped striations formed therein.

The cutting edge may be used to form the generally helically shaped striations in the peripheral wall of the optic if so desired (e g., to help reduce glare). Alternatively, the helical striations may be formed in the peripheral wall in a separate operation.

### Brief Description of the Drawing

Figure 1a is a side elevational, cross-sectional view of a preferred embodiment of the invention showing the IOL cutting mechanism in the ready position and the upper punch shown in spaced relation thereto;
Figure 1b is a cross-sectional view of the IOL cutting mechanism taken generally through the line 1b-1b of Figure 1a;
Figure 2 is a plan view of a prior art IOL;
Figure 3 a is the view of Fig. 1a with a portion of the IOL cutting mechanism shown in the raised position for retrieval of the cut IOL;
Figure 3b is a cross-sectional view of the IOL cutting mechanism taken generally through the line 3-3b of Figure 3a;
Figure 4a is a side elevational view of the trephine holder;
Figure 4b is a top plan view thereof;
Figure 4c is a cross-sectional view taken generally through the line 4c-4c of Figure 4b; Figure 5a is a side elevational view of the trephine blade;
Figure 5b is a top plan view thereof;
Figure 6a is a side elevational view of the lens pusher;
Figure 6b is a detail view of the lens-engaging end thereof;
Figure 6c is a top plan view thereof;
Figure 7 is a scanning electron microscope image at 50X magnification showing an IOL peripheral wall which was cut using the invention;
Figure 8a is a side elevational view of another embodiment of the invention with parts shown in cross-section; and
Figure 8b is a front elevational view of Figure 8a.

### Detailed Description of the Intention

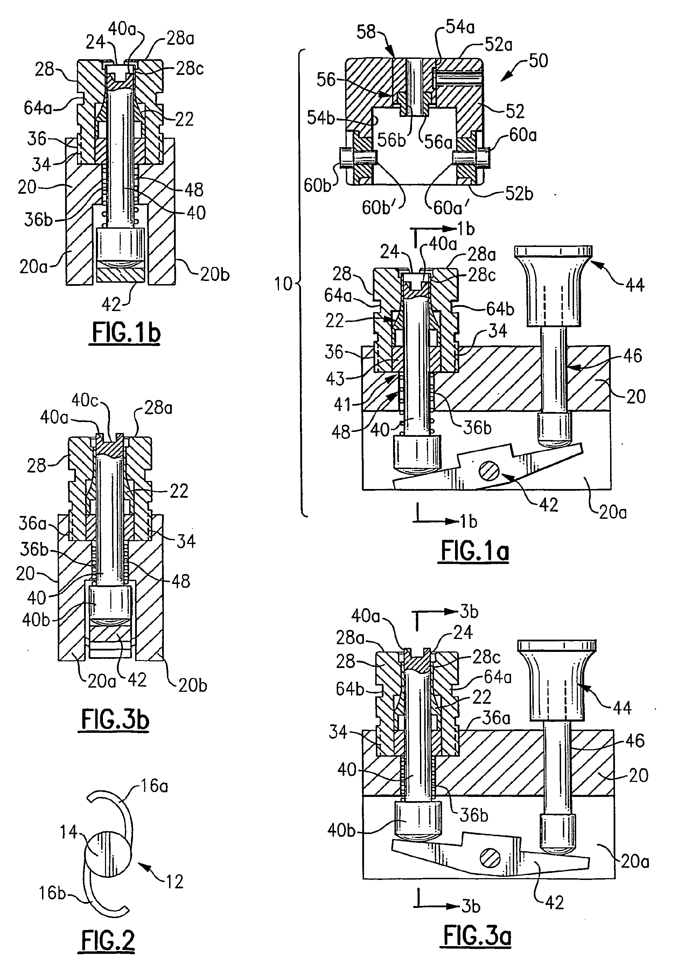

Referring now to the drawing, one embodiment of a fixture for cutting an IOL in accordance with the invention is shown and described, it being understood that other methods and fixtures for making an IOL in accordance with the invention is possible and within the scope of the invention. Thus, there is seen in Fig. 1a an IOL cutting fixture 10 useful for cutting an IOL optic from an IOL blank. A representative IOL 12 is seen in Fig. 2 to include an optic 14 and two haptics 16a and 16b. The optic 14 is provided to provide focusing within the eye while the haptics provide locating means for proper positioning of the IOL within the eye. It is understood that the particular configuration of IOL 12 is provided for discussion purposes only and may vary from that shown herein (e. g. , the IOL may include one or more haptics of any configuration and the IOL anterior and posterior optic surfaces may likewise vary). The invention is used for cutting the optic portion 14 of an IOL where the haptic portions are subsequently attached to the optic using any known means (e.g., gluing).

The IOL blank (not shown) from which the optic 14 is to be cut using the present invention is in any desired shape such as a sheet having any shape outline, for example. Most preferably, the IOL blank is provided in the shape of a. generally circular disc having anterior and posterior optical surfaces of the desired configuration (e.g., convey, concave or plano and may incorporate spherical and/or aspherical optics). The IOL blank itself may be molded using a metal mold, for example, and have the holes formed into the periphery for the subsequent staking of the haptics to the optic. Fixture 10 is therefore used for cutting a finished, square edge of the optic from the blank. Square edges at the periphery of an IOL optic have been clinically shown to help reduce the occurrence of posterior capsular opacification (PCO) or secondary cataracts as noted above.

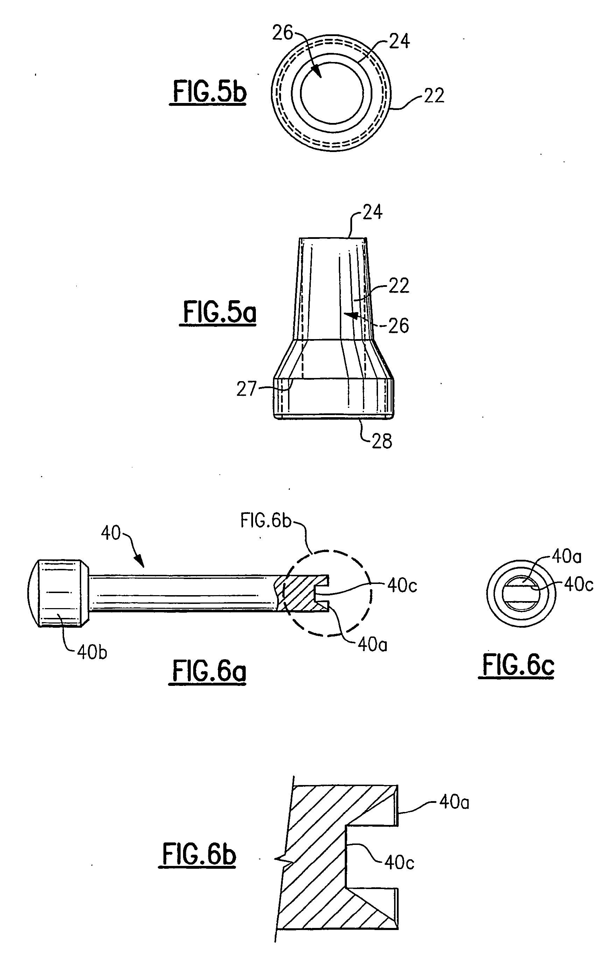

Fixture 10 includes a base 20 to which a trephine 22 is mounted having a circular cutting edge 24 at one end thereof and a longitudinally extending bore 26 extending entirely therethrough from cutting edge 24 to the opposite, base end 28 thereof (see also Figs. 5a,b). The diameter of the cutting edge 24 is selected to correspond to the desired resultant optic diameter (e.g., about 5-7mm, and more preferably about 6mm). The IOL blank diameter from which the finished optic is to be cut using fixture 10 is of course larger than the resultant cut diameter of the optic and may be in the range of about 7-9mm and more preferably is about 8mm in diameter.

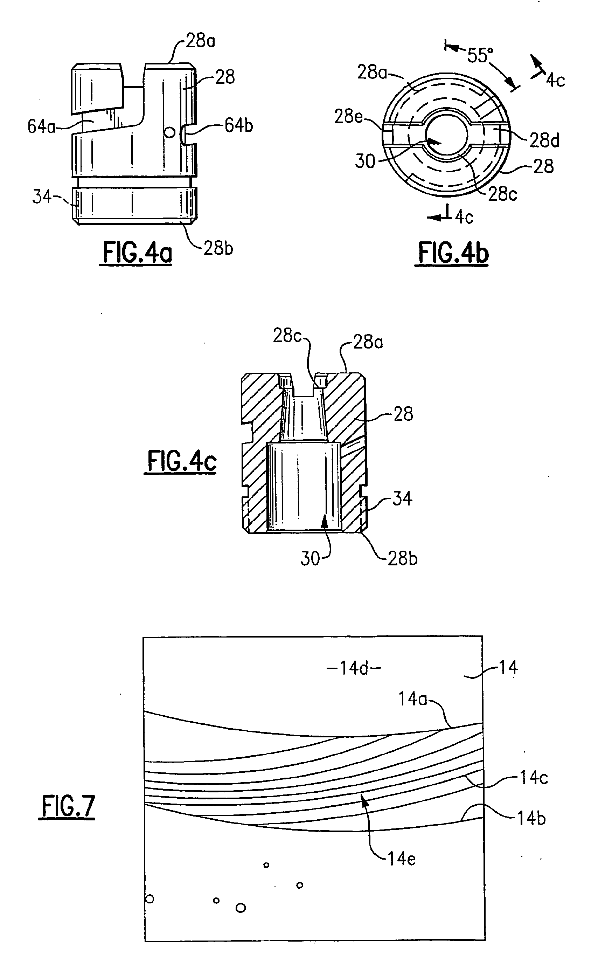

Trephine 22 is removably mounted within a trephine holder 28 having a longitudinal bore 30 extending entirely therethrough from top end 28a to bottom end 28b thereof (see also Figs. 4a-c). Bore 30 is sized and configured so that the trephine 22 may be inserted therein from bottom end 30 and come to rest at a position therein with the trephine cutting edge 24 located slightly above the counter-sunk top surface 28c. Trephine 22 and trephine holder 28 are removably mounted to base 20 via mating threads 34 formed adjacent the bottom end 28b of holder 28 and along the inner wall of a counter-sunk bore 36 formed in the top of base 20. Since the component parts are removably mounted to each other, the trephine 22 may be quickly exchanged for a new trephine when the cutting edge 24 thereof becomes dull or it is desired to switch to a different diameter cutting edge.

A lens pusher 40 is provided which extends through bore 36b and continues through the trephine central bore 26. The bottom end 40b of pusher 40 rests upon a rocker component 42 which itself is pivotally mounted between the spaced, parallel legs 20a and 20b of base 20. Lens pusher 40 is thus mounted for reciprocal longitudinal movement within trephine 22 and trephine holder 28. Accordingly, lens pusher 40 may be moved between the lowered position seen in Figs. 1a,b where the top end 40a thereof is located below the counter-sunk top surface 28c of trephine holder 28, to the raised position seen in Figs. 3a,3b where the top end 40a thereof is located above the counter-sunk top surface 28c of trephine holder 28. Reciprocal movement may be imparted by way of a pusher rod 44 which extends through another bore 46 formed in base 20 which extends parallel to bores 36a,b. The bottom end 44b of pusher rod 44 rests upon the end of rocker component 42 opposite lens pusher end 40b.

It is noted that lens pusher 40 is biased in the lowered position seen in Figs. 1a,1b by a spring 48 which surrounds the lens pusher shaft. The spring top end bears against the bottom surface 41 of bushing 43 (see Fig. 1a) and the spring bottom end bears against the ledge of the bottom end 40b of lens pusher 40. Thus, lens pusher 40 may be moved from the biased, lowered position to the raised position seen in Figs. 3a,b by simply pressing downwardly on pusher rod top end 44. Further explanation of this movement will be explained below.

Discussion is now turned to the upper punch mechanism 50 and the process by which an IOL optic is cut from an IOL blank. Referring to Fig. 1a, upper punch mechanism 50 is seen to include a main body portion 52 having a top surface 52a and a bottom end 52b with first and second, longitudinally extending bore portions 54a and 54b. Bottom bore portion 54b has an inner diameter slightly larger than the outer diameter of trephine holder 28 such that punch body portion 52 may be mounted upon trephine holder 28. A lens press insert 56 is mounted within upper bore portion 54a via an insert holder 58 which is slip-fit within bore 54a. The bottom edge 56a of lens press insert 56 is located at a position below upper bore portion 54a and within lower bore portion 54b. A pair of pins 60a,b are inserted via bearings 62a,b through the wall of body 52 with the pin ends 60a', 60b' extending radially into lower bore 54b. Pins 60a,b are preferably about 180° offset from each other. A pair of helically extending grooves 64a, 64b are formed in the outer surface of trephine holder 28 whereby pin ends 60a', 60b' may be inserted into the top end of the grooves adjacent top surface 28a (see Fig. 4a). The grooves first extend longitudinally toward bottom end 28b and then extend in a spiral pattern around the trephine holder body.

To begin the cutting process, a circular IOL blank is placed upon the trephine cutting edge 24 which is located slightly above the counter-sunk surface 28c of trephine holder 28 yet below the trephine holder upper surface 28a. The upper surface 28a defines a circular counter-sunk surface 28c as seen best in Fig. 4b. The outer diameter of the counter-sunk surface 28c is sized to approximate the diameter of the IOL blank being placed therein such that the IOL blank becomes centered on the trephine cutting edge 24. With the IOL blank resting on trephine cutting edge 24, the upper punch body 52 is lowered onto the trephine holder 28 with pin ends 60a', 60b' aligned with respective grooves 64a, 64b formed in holder 28. Since the grooves first extend longitudinally toward holder bottom edge 28b, the punch body 52 will translate linearly in a telescoping movement onto trephine holder 28. Upon reaching the end of the longitudinally extending section of the grooves, the lens press insert bottom edge 56a rests lightly upon the IOL blank. The operator then rotates punch body 52 with pin ends 60a', 60b' riding along the helical extents of grooves 64a, 64b whereby the lens press insert 56 pushes against the IOL blank, forcing it into the trephine cutting edge 2 4 which itself remains stationary. It is understood, however, that variations in operation may be made so that the trephine instead moves into the IOL blank which is held stationary. The trephine and IOL blank may also move together into one another if desired.

It will be realized that the above-described rotation of the punch body 52 relative to the trephine holder 28 causes the trephine cutting edge 24 to cut through the IOL blank. It is noted that the lens press insert 56 includes a longitudinal bore 56b extending therethrough. This is provided so that the central optical surface of the IOL blank is not touched by the lens press insert which could potentially cause harm to the optical surface. The diameter of the lens press insert bottom edge 56a is sized to so that the IOL blank is sandwiched between the insert and the trephine cutting blade 24. Once the IOL blank has been cut, the punch body is rotated in the opposite direction and removed from the trephine holder 28, leaving the cut IOL optic resting on the trephine cutting blade 24. The annular flash which has been cut from the optic is located around the cutting edge 24 on counter-sunk surface 28c. To remove the flash, the operator uses tweezers, extending them within either radial relief 28d or 28e formed in the top surface of the trephine holder 28 (see Fig. 4b). With the flash removed, the operator moves the lens pusher 40 to the raised position by pressing downwardly on pusher rod 44 as explained above. With the cut IOL optic resting on the top end 40a of pusher 40, the IOL optic is readily accessible for retrieval thereof. The operator may thus retrieve the cut IOL optic using a pair of tweezers, for example, by extending the tweezer tips through the relief 40c formed in top end 40a (see Figs. 6a-c).

Attention is turned to Fig. 7 which is a scanning electron microscope image of an IOL optic cut using the present invention. The resultant peripheral wall 14c is defined between anterior and posterior peripheral edges 14a, 14b which are located at the junctures of the anterior optic surface 14d and opposite posterior optic surface, respectively (not shown). Helical striations 14e are seen in peripheral wall 14c which are a result of the rotational movement of the trephine cutting edge 24. These striations 14e may contribute to a decrease in unwanted glare caused by reflection of light off the edge of the implanted optic.

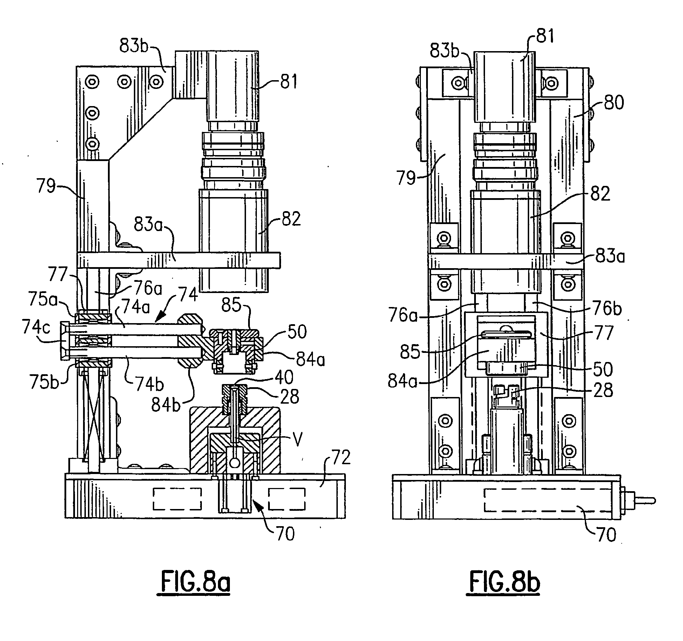

Referring now to Figures 8a and 8b, another embodiment of the invention is shown which incorporates various operating efficiencies to the invention. As will be described below, there are several different aspects for increasing operating efficiency of the invention, however, it is understood that any number (including none) of the different operating efficiencies may be employed depending on the desires of the user.

Thus, in a first aspect of operational efficiency, movement of the lens pusher 40 may be automated via a pneumatic cylinder 70 mounted within support base 72 and connected to cause lens pusher 40 to linearly translate in the intended manner. A button or other actuator (not shown) is engaged (either manually or via automated controls) to alternately activate and deactivate the cylinder 70 causing the reciprocal linear movement of the lens pusher 40 as described previously.

In a second aspect of operational efficiency, a vacuum line V may be incorporated longitudinally through the center of lens pusher 40 to assist in maintaining the IOL blank and/or optic on the lens pusher 40 until it is time to remove the IOL therefrom, at which time the vacuum V is released. This is a particularly useful feature for performing secondary processes on the IOL blank and/or cut optic. For example, some IOLs are made with two or more holes formed in the periphery of the optic. After the optic has been cut, one end of each of two or more haptics is secured (e.g., with an adhesive) within a respective hole in the optic. Due to the precision necessary to insert the haptic end into a respective hole, it is necessary to maintain the optic stationary during the haptic attachment process. The vacuum secures the optic in place upon the lens pusher 40 while inserting (e.g., gluing) the haptics into the holes formed in the cut optic periphery. In the past, this has been a separate process step in the IOL manufacturing process. This step may now be incorporated into this process station (i.e., the process that cuts the square edge on the IOL optic) and the efficiency of the overall manufacturing process is thereby increased.

In a third aspect of operational efficiency, the upper punch mechanism 50 is mounted to a support arm 74 extending generally horizontally from vertical arms 76a, 76b. Support arm 74 may include two spaced, parallel arms 74a and 74b which attach to ball bushings 75a, 75b which themselves are mounted within a vertical slide block 77 (Fig. 8b). Vertical slide block 77 is mounted to spaced, parallel vertical arms 76a, 76b which extend between vertical frames 79, 80. The vertical slide block 77 and ball bushings 75a, 75b permit the alternate raising and lowering of arms 74a, 74b, together with upper punch mechanism 50, upon vertical arms 76a, 76b with respect to stationary trephine holder 28. This permits the operator to easily and quickly remove and attach the upper punch mechanism 50 to the trephine holder 28 between sequential IOL cutting operations as described previously. It is also possible to use automated controls to effectuate this reciprocal vertical movement of mechanism 50, if desired.

When it is time to cut the IOL blank located on lens pusher 40, the upper punch mechanism 50 is lowered to sit upon trephine holder 28 as described above. In order to provide the necessary rotational movement of mechanism 50 upon trephine holder 28 to cut the IOL blank, mechanism 50 is slip fit within a bushing 84a which itself is mounted to arms 74a, 74b via mounting bracket 84b. A circular cap 85 is attached to mechanism 50 and has a diameter which is larger than the central aperture of bushing 84a such that the cap rests on the top surface of the bushing 84a as seen clearly in Fig. 8a. With mechanism 50 lowered and resting upon trephine holder 28, an operator (or other suitable automated mechanism) may turn cap 85 which will in turn rotate mechanism 50 to effectuate the rotational, linear movement of the mechanism 50 upon trephine holder 28 as described previously, and thereby cutting the square edge into the IOL blank. Once the IOL has been cut, the cap 85 is turned in the opposite direction to raise it and mechanism 50 above trephine holder 28.

It is noted that the support arms 74a, 74b may also be selectively translated rearwardly along a generally horizontal plane toward vertical arms 76a, 76b by virtue of ball bushings 75a, 75b through which the arms 76a, 76b completely extend, terminating in a common end plate 74c (Fig. 8a). This feature provides clearance of the upper punch mechanism 50 from the trephine holder 28 when needed (e.g., during haptic insertion described above and inspection as described below). It is noted that other movements may be imparted to the fixture to permit this clearance, e.g., a pivoting of arms 76a, 76b about the vertical mounts.

In a fourth aspect of operational efficiency, a CCD camera 81 and focusing lens 82 are provided on vertical mounts 79, 80 (e.g., via brackets 83a,b) in a position directly above upper punch mechanism 50. A monitor (not shown) may be attached to camera 81 and lens 82 to allow an operator a clear, magnified view of the working area, particularly the IOL optic during the haptic insertion process.

The above described operating efficiencies offer a number of advantages including, for example, better viewing of the IOL blank to improve centering on the lens pusher, decrease of worker eye fatigue during haptic insertion, reduction in the number of different processing stations and thus a reduction in the amount of IOL handling, and reduced labor costs.

## Claims

1. A method for cutting an IOL optic having opposite anterior (14d) and posterior surfaces and a peripheral wall (14e) extending therebetween out of an IOL blank, said method comprising the steps of:
a) providing a generally circular cutting edge (24);
b) providing a lens press (56) having a generally circular lens-engaging end (56a);
c) positioning said IOL blank between said generally circular cutting edge (24) and said generally circular lens-engaging end (56a) of said lens press (56); **characterized in that**,
d) moving said lens press (56) and said cutting edge (24) toward one another in a rotational translation with said cutting edge (24) rotationally cutting through said IOL blank and thereby forming said IOL optic.

2. The method of claim 1 wherein said generally circular cutting edge (24) is defined on one end of a trephine (22).

3. The method of claim 2 wherein said IOL blank is generally circular having a diameter of between about 7 to 9mm and said cut IOL optic has a diameter of between about 5 to 7mm.

4. The method of claim 3 and further including the step of providing a trephine holder (28) having a generally circular counter-sunk surface (28c) wherein said cutting edge (24) is located and said IOL blank is centered prior to cutting.

5. The method of claim 4 and further providing the step of providing a lens pusher (40) having a lens-engaging end (40a) and extending coaxially through said trephine (22), said lens pusher (40) being movable between raised and lowered positions wherein said lens-engaging end (40a) of said lens pusher (40) is positioned above and below said cutting edge (24), respectively.

6. The method of claim 5 and further comprising the step of biasing said lens pusher (40) in the lowered position.

7. The method of claim 6 and further comprising the step of providing an upper punch body (52) wherein said tens press (56) is located, said upper punch body (52) being removably mountable upon said trephine holder (28).

8. The method of claim 7 wherein said upper punch body (52) has a longitudinally extending bore (54b) and includes one or more pins (60a, 60b) extending radially into said bore (54b), and wherein said trephine holder (28) includes one or more grooves (64a, 64b) which align and engage with said one or more pins (60a, 60b) to perform said rotational cutting movement.

9. Apparatus for cutting an IOL optic having opposite anterior (14d) and posterior surfaces and a peripheral wall (14e) extending therebetween out of an IOL blank, said apparatus comprising:
a) a generally circular cutting edge (24);
b) a lens press (56) having a generally circular lens-engaging end (56a) with said IOL blank positionable between said generally circular cutting edge (54) and said generally circular lens-engaging end (56a) of said lens press (56);
**characterized in that**, said IOL optic is formed by moving said lens press (56) and said cutting edge (24) toward one another in a rotational translation with said cutting edge rotationally cutting through said IOL blank and thereby forming said IOL optic.

10. The apparatus of claim 9 wherein said generally circular cutting edge (24) is defined on one end of a trephine (22).

11. The apparatus of claim 9 wherein said lens press (56) is mounted to permit selective movement thereof into and out of alignment with said generally circular cutting edge (24).

12. The apparatus of claim 11 and further comprising a CCD camera (81) and focusing lens (82) mounted to permit viewing of said IOL blank and cut IOL optic.

13. The apparatus of claim 10 wherein said IOL blank is generally circular having a diameter of between about 7 to 9mm and said cut IOL optic has a diameter of between about 5 to 7mm.

14. The apparatus of claim 13 and further comprising a trephine holder (28) having a circular counter-sunk surface (28c) wherein said cutting edge (24) is located and said IOL blank is centered prior to cutting.

15. The apparatus of claim 14 and further comprising a lens pusher (40) having a lens-engaging end (40a) and extending coaxially through said trephine (22), said lens pusher (40) being movable between raised and lowered positions wherein said lens-engaging end (40a) of said lens pusher (40) is positioned above and below said cutting edge (24), respectively.

16. The apparatus of claim 15 and further comprising means (48) biasing said lens pusher (40) in the lowered position.

17. The apparatus of claim 16 and further comprising an upper punch body (52) wherein said lens press (56) is located, said upper punch body (52) being removably mountable upon said trephine holder (28).

18. The apparatus of claim 1 wherein said upper punch body (52) has a longitudinally extending bore (54b) and includes one or more pins (60a, 60b) extending radially into said bore (54b), and wherein said trephine holder (28) includes one or more grooves (64a, 64b) which align and engage with said one or more pins (60a, 60b) to perform said rotational cutting movement.

19. The method of claim 1, further comprising the step of forming generally helically shaped striations in said peripheral wall.

20. The apparatus of claim 9, adapted such that the cutting edge will form generally helically shaped striations in said peripheral wall.

## Patentansprüche

1. Verfahren zum Schneiden einer IOL-Optik mit gegenüberliegenden anterioren (14d) und posterioren Oberflächen und einer sich dazwischen erstreckenden Umfangswand (14e) aus einem IOL-Rohling, wobei das Verfahren die Schritte aufweist:
a) Bereitstellen einer im wesentlichen kreisförmigen Schneidkante (24);
b) Bereitstellen einer Linsenpresse (56) mit einem im wesentlichen kreisförmigen Linseneingriffsende (56a);
c) Anordnen des IOL-Rohlings zwischen der im wesentlichen kreisförmigen Schneidkante (24) und dem im wesentlichen kreisförmigen Linseneingriffsende (56a) der Linsenpresse (56);
**gekennzeichnet durch**
d) Bewegen der Linsenpresse (56) und der Schneidkante (24) zueinander in einer Drehtranslation, wobei sich die Schneidkante (24) drehend **durch** den IOL-Rohling schneidet und **dadurch** die IOL-Optik bildet.

2. Verfahren nach Anspruch 1, wobei die im wesentlichen kreisförmige Schneidkante (24) an einem Ende eines Trepans (22) definiert ist.

3. Verfahren nach Anspruch 2, wobei der IOL-Rohling im wesentlichen kreisförmig ist, wobei er einen Durchmesser zwischen etwa 7 bis 9 mm aufweist und die ausgeschnittene IOL-Optik einen Durchmesser zwischen etwa 5 bis 7 mm aufweist.

4. Verfahren nach Anspruch 3, das ferner den Schritt des Bereitstellens eines Trepanhalters (28) mit einer im wesentlichen kreisförmigen versenkten Oberfläche (28c) aufweist, in der sich die Schneidkante (24) befindet und der IOL-Rohling vor dem Schneiden zentriert wird.

5. Verfahren nach Anspruch 4, das ferner den Schritt des Bereitstellens eines Linsenschiebers (40) aufweist, der ein Linseneingriffsende (40a) aufweist und sich koaxial durch den Trepan (22) erstreckt, wobei der Linsenschieber (40) zwischen angehobenen und abgesenkten Positionen beweglich ist, wobei das Linseneingriffsende (40a) des Linsenschiebers (40) über bzw. unter der Schneidkante (24) angeordnet ist.

6. Verfahren nach Anspruch 5, das ferner den Schritt des Vorspannens des Linsenschiebers (40) in die abgesenkte Position aufweist.

7. Verfahren nach Anspruch 6, das ferner den Schritt des Bereitstellens eines oberen Stanzkörpers (52) aufweist, in dem sich die Linsenpresse (56) befindet, wobei der obere Stanzkörper (52) abnehmbar auf dem Trepanhalter (28) anbringbar ist.

8. Verfahren nach Anspruch 7, wobei der obere Stanzkörper (52) eine sich longitudinal erstreckende Bohrung (54b) aufweist und einen oder mehrere Stifte (60a, 60b) aufweist, die sich radial in die Bohrung (54b) erstrecken, und wobei der Trepanhalter (28) eine oder mehrere Nuten (64a, 64b) aufweist, die mit dem einen oder den mehreren Stiften (60a, 60b) ausgerichtet sind und sie in Eingriff nehmen, um die Drehschneidbewegung durchzuführen.

9. Vorrichtung zum Schneiden einer IOL-Optik mit gegenüberliegenden anterioren (14d) und posterioren Oberflächen und einer sich dazwischen erstreckenden Umfangswand (14e) aus einem IOL-Rohling, wobei die Vorrichtung aufweist:
a) eine im wesentlichen kreisförmige Schneidkante (24);
b) eine Linsenpresse (56) mit einem im wesentlichen kreisförmigen Linseneingriffsende (56a), wobei der IOL-Rohling zwischen der im wesentlichen kreisförmigen Schneidkante (54) und dem im wesentlichen kreisförmigen Linseneingriffsende (56a) der Linsenpresse (56) positionierbar ist;
**dadurch gekennzeichnet, daß** die IOL-Optik durch Bewegen der Linsenpresse (56) und der Schneidkante (24) zueinander in einer Drehtranslation gebildet wird, wobei sich die Schneidkante drehend durch den IOL-Rohling schneidet und **dadurch** die IOL-Optik bildet.

10. Vorrichtung nach Anspruch 9, wobei die im wesentlichen kreisförmige Schneidkante (24) an einem Ende eines Trepans (22) definiert ist.

11. Vorrichtung nach Anspruch 9, wobei die Linsenpresse (56) so angebracht ist, daß deren selektive Bewegung in und aus einer Ausrichtung mit der im wesentlichen kreisförmigen Schneidkante (24) ermöglicht wird.

12. Vorrichtung nach Anspruch 11, die ferner eine CCD-Kamera (81) und eine Fokussierungslinse (82) aufweist, die so angebracht sind, daß eine Betrachtung des IOL-Rohlings und der geschnittenen IOL-Optik ermöglicht wird.

13. Vorrichtung nach Anspruch 10, wobei der IOL-Rohling im wesentlichen kreisförmig ist, wobei er einen Durchmesser zwischen etwa 7 bis 9 mm aufweist, und die geschnittene IOL-Optik einen Durchmesser zwischen etwa 5 bis 7 mm aufweist.

14. Vorrichtung nach Anspruch 13, die ferner einen Trepanhalter (28) mit einer kreisförmigen versenkten Oberfläche (28c) aufweist, in der sich die Schneidkante (24) befindet und der IOL-Rohling vor dem Schneiden zentriert wird.

15. Vorrichtung nach Anspruch 14, die ferner einen Linsenschieber (40) aufweist, der ein Linseneingriffsende (40a) aufweist und sich koaxial durch den Trepan (22) erstreckt, wobei der Linsenschieber (40) zwischen angehobenen und abgesenkten Positionen beweglich ist, wobei das Linseneingriffsende (40a) des Linsenschiebers (40) über bzw. unter der Schneidkante (24) angeordnet ist.

16. Vorrichtung nach Anspruch 15, die ferner eine Einrichtung (48) aufweist, die den Linsenschieber (40) in die abgesenkte Position vorspannt.

17. Vorrichtung nach Anspruch 16, die ferner einen oberen Stanzkörper (52) aufweist, in dem sich die Linsenpresse (56) befindet, wobei der obere Stanzkörper (52) abnehmbar auf dem Trepanhalter (28) anbringbar ist.

18. Vorrichtung nach Anspruch 17, wobei der obere Stanzkörper (52) eine sich longitudinal erstreckende Bohrung (54b) aufweist und einen oder mehrere Stifte (60a, 60b) aufweist, die sich radial in die Bohrung (54b) erstrecken, und wobei der Trepanhalter (28) eine oder mehrere Nuten (64a, 64b) aufweist, die mit dem einen oder den mehreren Stiften (60a, 60b) ausgerichtet sind und sie in Eingriff nehmen, um die Drehschneidbewegung durchzuführen.

19. Verfahren nach Anspruch 1, das ferner den Schritt des Bildens von im wesentlichen schraubenförmigen Rillen in der Umfangswand aufweist.

20. Vorrichtung nach Anspruch 9, die so eingerichtet ist, daß die Schneidkante im wesentlichen schraubenförmige Rillen in der Umfangswand bilden wird.

## Revendications

1. Procédé d'usinage d'une optique de lentille intra-oculaire comportant des surfaces antérieure (14d) et postérieure opposées et une paroi périphérique (14e) s'étendant entre elles à partir d'une pièce brute de lentille intra-oculaire, ledit procédé comprenant les étapes consistant à:
a) prévoir une arête tranchante (24) généralement circulaire ;
b) prévoir une presse à lentilles (56) comportant une extrémité de mise en prise avec une lentille (56a) généralement circulaire ;
c) positionner ladite pièce brute de lentille intra-oculaire entre ladite arête tranchante (24) généralement circulaire et ladite extrémité de mise en prise avec une lentille (56a) généralement circulaire de ladite presse à lentilles (56),
**caractérisé par**
d) le déplacement de ladite presse à lentilles (56) et de ladite arête tranchante (24) l'une vers l'autre dans une translation en rotation, ladite arête tranchante (24) usinant en rotation ladite pièce brute de lentille intra-oculaire et formant de ce fait ladite optique de lentille intra-oculaire.

2. Procédé selon la revendication 1, dans lequel ladite arête tranchante (24) généralement circulaire est définie sur une extrémité d'un trépan (22).

3. Procédé selon la revendication 2, dans lequel ladite pièce brute de lentille intra-oculaire est généralement circulaire, avec un diamètre entre environ 7 et 9 mm, et ladite optique de lentille intra-oculaire usinée a un diamètre entre environ 5 et 7 mm.

4. Procédé selon la revendication 3 et comprenant en outre l'étape consistant à prévoir un support de trépan (28) comportant une surface de lamage (28c) généralement circulaire dans laquelle ladite arête tranchante (24) est située et ladite pièce brute de lentille intra-oculaire est centrée avant l'usinage.

5. Procédé selon la revendication 4 et comprenant en outre l'étape consistant à prévoir un pousseur de lentille (40) comportant une extrémité de mise en prise avec une lentille (40a) et s'étendant coaxialement à travers ledit trépan (22), ledit pousseur de lentille (40) pouvant être déplacé entre des positions élevée et abaissée auxquelles ladite extrémité de mise en prise avec une lentille (40a) dudit pousseur de lentille (40) est positionnée au-dessus et au-dessous de ladite arête tranchante (24), respectivement.

6. Procédé selon la revendication 5 et comprenant en outre l'étape consistant à solliciter ledit pousseur de lentille (40) à la position abaissée.

7. Procédé selon la revendication 6 et comprenant en outre l'étape consistant à prévoir un corps d'emporte-pièce supérieur (52) dans lequel ladite presse à lentilles (56) est située, ledit corps d'emporte-pièce supérieur (52) pouvant être monté de manière amovible sur ledit support de trépan (28).

8. Procédé selon la revendication 7, dans lequel ledit corps d'emporte-pièce supérieur (52) comporte un alésage (54b) s'étendant longitudinalement et comprend une ou plusieurs broches (60a, 60b) s'étendant radialement dans ledit alésage (54b), et dans lequel ledit support de trépan (28) comprend une ou plusieurs rainures (64a, 64b) qui s'alignent et viennent en prise avec lesdites une ou plusieurs broches (60a, 60b) pour effectuer ledit mouvement d'usinage en rotation.

9. Appareil pour usiner une optique de lentille intra-oculaire comportant des surfaces antérieure (14d) et postérieure opposées et une paroi périphérique (14e) s'étendant entre elles à partir d'une pièce brute de lentille intra-oculaire, ledit appareil comprenant :
a) une arête tranchante (24) généralement circulaire ;
b) une presse à lentilles (56) comportant une extrémité de mise en prise avec une lentille (56a) généralement circulaire, ladite pièce brute de lentille intra-oculaire pouvant être positionnée entre ladite arête tranchante (24) généralement circulaire et ladite extrémité de mise en prise avec une lentille (56a) généralement circulaire de ladite presse à lentilles (56),
**caractérisé en ce que** ladite optique de lentille intra-oculaire est formée en déplaçant ladite presse à lentilles (56) et ladite arête tranchante (24) l'une vers l'autre dans une translation en rotation, ladite arête tranchante usinant en rotation ladite pièce brute de lentille intra-oculaire et formant de ce fait ladite optique de lentille intra-oculaire.

10. Appareil selon la revendication 9, dans lequel ladite arête tranchante (24) généralement circulaire est définie sur une extrémité d'un trépan (22).

11. Appareil selon la revendication 9, dans lequel ladite presse à lentilles (56) est montée pour permettre un mouvement sélectif de celle-ci en alignement et hors d'alignement avec ladite arête tranchante (24) généralement circulaire.

12. Appareil selon la revendication 11 et comprenant en outre une caméra à dispositif à transfert de charge (81) et une lentille de mise au point (82) montées pour permettre de voir ladite pièce brute de lentille intra-oculaire et d'usiner l'optique de lentille intra-oculaire.

13. Appareil selon la revendication 10, dans lequel ladite pièce brute de lentille intra-oculaire est généralement circulaire, avec un diamètre entre environ 7 et 9 mm, et ladite optique de lentille intra-oculaire a un diamètre entre environ 5 et 7 mm.

14. Appareil selon la revendication 13 et comprenant en outre un support de trépan (28) comportant une surface de lamage (28c) circulaire dans laquelle ladite arête tranchante (24) est située et ladite pièce brute de lentille intra-oculaire est centrée avant l'usinage.

15. Appareil selon la revendication 14 et comprenant en outre un pousseur de lentille (40) comportant une extrémité de mise en prise avec une lentille (40a) et s'étendant coaxialement à travers ledit trépan (22), ledit pousseur de lentille (40) pouvant être déplacé entre des positions élevée et abaissée auxquelles ladite extrémité de mise en prise avec une lentille (40a) dudit pousseur de lentille (40) est positionnée au-dessus et au-dessous de ladite arête tranchante (24), respectivement.

16. Appareil selon la revendication 15 et comprenant en outre des moyens (48) sollicitant ledit pousseur de lentille (40) à la position abaissée.

17. Appareil selon la revendication 16 et comprenant en outre un corps d'emporte-pièce supérieur (52) dans lequel ladite presse à lentilles (56) est située, ledit corps d'emporte-pièce supérieur (52) pouvant être monté de manière amovible sur ledit support de trépan (28).

18. Appareil selon la revendication 17, dans lequel ledit corps d'emporte-pièce supérieur (52) comporte un alésage (54b) s'étendant longitudinalement et comprend une ou plusieurs broches (60a, 60b) s'étendant radialement dans ledit alésage (54b), et dans lequel ledit support de trépan (28) comprend une ou plusieurs rainures (64a, 64b) qui s'alignent et viennent en prise avec lesdites une ou plusieurs broches (60a, 60b) pour effectuer ledit mouvement d'usinage en rotation.

19. Procédé selon la revendication 1, comprenant en outre l'étape consistant à former des striations de forme généralement hélicoïdale dans ladite paroi périphérique.

20. Appareil selon la revendication 9, adapté pour que l'arête tranchante forme des striations de forme généralement hélicoïdale dans ladite paroi périphérique.
